# EUROPEAN PATENT APPLICATION

(11) **EP 4 219 565 A1**
(43) Date of publication of application: **02.08.2023**
(21) Application number: 23161874.5
(22) Date of filing: 05.06.2015
(51) Int. Cl.: C07K 16/46, A61K 39/395, A61P 37/06, A61K 47/60, A61K 47/68, A61K 38/28, A61K 38/26

(54) **METHOD FOR DECREASING IMMUNOGENICITY OF PROTEIN AND PEPTIDE**

(30) Priority: 05.06.2014 KR 20140068660
(62) Divisional of application: 15802343.2
(71) Applicant: Hanmi Pharm. Co., Ltd., Hwaseong-si, Gyeonggi-do 445-958 (KR)
(72) Inventor: PARK, Sung Hee, 463-959 Gyeonggi-do (KR); KIM, Seung Su, 137-953 Seoul (KR); LIM, Hyung Kyu, 445-996 Gyeonggi-do (KR); CHOI, Jae Hyuk, 138-780 Seoul (KR); CHOI, In Young, 448-160 Gyeonggi-do (KR); KWON, Se Chang, 135-506 Seoul (KR)
(74) Representative: Mewburn Ellis LLP

(57) **Abstract**

The present invention relates to a method for increasing serum half-life of a protein or peptide and decreasing immunogenicity thereof by site-specifically binding a carrier to a protein or peptide, and to the use thereof. The conjugate of the physiologically active protein or peptide of the present invention can significantly decrease immunogenicity in the human body and thus reduce antibody production rate against the protein or peptide. Therefore, the present conjugate has advantages in that a phenomenon of reduced clinical effects of the physiologically active protein or peptide is low, and it can be effectively used in the development of long-acting formulations having a high safety against the immune response.

## Description

### Technical Field

The present invention relates to a method for increasing serum half-life of a protein or peptide and decreasing immunogenicity thereof by site-specifically binding a carrier to a protein or peptide, and to the use thereof.

### Background Art

Immune responses to biological therapeutic agents can be widely induced for both non-human and human-derived proteins. These responses may weaken clinical effects, limit the efficacy, and sometimes lead to pathological diseases or even cause the death of the patient. In particular, the production of neutralizing antibodies that target the recombinant self-protein may induce a cross-reaction with the protein inherent in the body of the patient and thus lead to serious consequences (see, Lim LC. Hematology 2005 10(3):255-9). Problems of biopharmaceuticals such as monoclonal antibodies were greatly reduced with the development of molecular biology. However, many recombinant protein pharmaceuticals are identical with the protein sequences which are expressed in the body and thus, there still remain a possibility of causing a neutralizing immune response (see, Namaka M et al, Curr Med Res Opin 2006 22(2):223-39). Although the mechanism by which it is possible to induce immunogenicity is not wholly clear, it is known that the resistance to self-proteins can be broken by products administered to the patient and by various factors of the patient (reviewed in Chester, K, Baker, MP and Mayer A. Expert Rev Clin Immunol 2005 1(4): 549-559, Baker MP and Jones TD. Curr. Opin. Drug Disc Dev 2007 10(2):219-227). Factors for immunogenicity include dosage, frequency and route of administration, immunomodulatory ability of protein drugs, their preparation and the like. The most important factor to induce the immune response is whether there is an antigen recognition site (epitope) that can effectively stimulate a CD4 + T cell response (reviewed Baker MP and Jones TD. Curr. Opin. Drug Disc Dev 2007 10(2):219-227).

On the other hand, exendin-4 is a natural peptide discovered in the salivary gland of the Gila monster lizard and has a 52% sequence similarity with human GLP-1 (glucagon-like peptide-1). Exendin-4 and GLP-1 have a similar insulin secretion function. However, GLP-1 is rapidly deactivated by dipeptidyl peptidase-IV (DPP-IV), thus having a very short half-life, whereas exendin-4 keeps the resistance to DPP-IV by glycine being present instead of alanine in the second amino acid sequence and thus can be more effective as a therapeutic agent of type II diabetes. In addition, insulin or analogs thereof, and dual agonists of GLP-1/glucagon are also used as therapeutic agents for diabetes and obesity. However, the presence of these non-human amino acid sequences can act as an antigen recognition site of T cells. Exenatide (Byetta) which was approved as therapeutic agents of type II diabetes as synthetic exendin-4 has produced an antibody to exenatide for more than 30% of patients who received administration of exenatide for one year in clinical trials. Lixisenatide, approved recently, has produced an antibody for about 60-71% of patients (see, Zinman, B. et al., Annals of Internal Medicines. 2007 146(7): 477-486; Schnabel CA et al, Peptides 2006 27:1902-1910; DeFronzo, R.A. et al, Diabetes Care 2005 28:1092-1100; Buse, J.B. et al, Diabetes Care 2004 27:2628-2635). That is, exentide was recognized as an *in* vivo extraneous substance to be treated and the antibody was produced. For this reason, the problem that is difficult to reliably expect a therapeutic effect is increasing prevalent.

Therefore, in the case of a physiologically active protein or peptide which has been administered within the body for the purpose of treatment or prevention for a long period of time, it is important to control the immunogenicity. In particular, adult disease-related physiologically active proteins or peptides such as insulin or insulinotropic peptide and anti-obesity protein have often been developed as as long-acting formulations capable of lasting in the body after administration. In addition, even if they are not long-acting formulations, there are many cases in which they must be administered several times for a long period of time. Therefore, not inducing an immune response is an important issue.

Under these circumstances, the present inventors have conducted numerous studies and experiments to develop pharmaceutical formulations of a protein or peptide which do not induce an immune response. As a result, the inventors have discovered that, when a carrier site-specifically binds to a protein or peptide, the immunogenicity can be decreased as compared to that of a protein or peptide to which the carrier has not been bound, thus completing the present invention.

### Disclosure of Invention

### Technical Problem

One object of the present inveniton is to provide a method for decreasing immunogenicity of physiologically active proteins or peptides.

Another object of the present invention is to provide a composition, comprising a conjugate of a physiologically active protein or peptide in which a carrier is bound to the non-terminal, internal residue of a physiologically active protein or peptide, via a non-peptidyl linker.

Another object of the present invention is to provide a method for preparing the conjugate of the physiologically active protein or peptide, in which the carrier is bound to the non-terminal, internal residue of the physiologically active protein or peptide.

### Solution to Problem

In one aspect, the present invention provides a method for decreasing immunogenicity of a physiologically active protein or peptide as compared to that of a physiologically active protein or peptide to which a carrier is not bound, which comprises binding a carrier to the non-terminal, internal residue of the physiologically active protein or peptide.

In one specific embodiment of the invention, the above carrier is characterized in that it is selected from the group consisting of a polyethylene glycol, a fatty acid, a cholesterol, an albumin or a fragment thereof, an albumin-binding substance, a polymer having repeating units of a particular amino acid sequence, an antibody, an antibody fragment, an FcRn binding substance, an in-vivo connective tissue or a derivative thereof, a nucleotide, a fibronectin, a transferrin, an elastin-like polypeptide(ELP), an XTEN polypeptide, a carboxy-terminal peptide (CTP), a structure inducing probe (SIP), a saccharide and a high molecular weight polymer.

In another specific embodiment of the invention, the FcRn binding substance is characterized in that it includes an immunoglobulin Fc region.

In another specific embodiment of the invention, the physiologically active protein or peptide and the carrier are characterized by being bound via a linker interposed therebetween.

In another specific embodiment of the invention, the linker is characterized in that it is a non-peptidyl linker.

In another specific embodiment of the invention, the non-peptidyl linker is characterized in that it is selected from the group consisting of a polyethylene glycol, a polypropylene glycol, an ethylene glycol-propylene glycol copolymer, a polyoxyethylated polyol, a polyvinyl alcohol, a polysaccharide, a dextran, a polyvinyl ethyl ether, a biodegradable polymer, a lipid polymer, a chitin, a hyaluronic acid and a combination thereof.

In another specific embodiment of the invention, it is characterized in that the physiologically active protein or peptide is bound to an immunoglobulin Fc region via a non-peptidyl polymer which is selected from the group consisting of a polyethylene glycol, a polypropylene glycol, an ethylene glycol-propylene glycol copolymer, a polyoxyethylated polyol, a polyvinyl alcohol, a polysaccharide, a dextran, a polyvinyl ethyl ether, a biodegradable polymer, a lipid polymer, a chitin, a hyaluronic acid and a combination thereof.

In another specific embodiment of the invention, the physiologically active protein or peptide is characterized in that it is selected from the group consisting of an anti-obesity peptide, an insulinotropic peptide or an analog thereof, a leptin, an insulin, an insulin analog, a glucagon, a human growth hormone, a growth hormone releasing hormone, a growth hormone releasing peptide, an interferon, an interferon receptor, a colony stimulating factor, a glucagon-like peptide such as GLP-1, a GLP-1/glucagon dual agonist, a gastric inhibitory polypeptide (GIP), a G-protein-coupled receptor, an interleukin, an interleukin receptor, an enzyme, an interleukin binding protein, a cytokine binding protein, a macrophage activating factor, a macrophage peptide, a B cell factor, a T cell factor, a protein A, an allergy inhibitory factor, a cell necrosis glycoprotein, an immunotoxin, a lymphotoxin, a tumor necrosis factor, a tumor inhibitory factor, a metastasis growth factor, an alpha-1 antitrypsin, an albumin, an α-lactalbumin, an apolipoprotein-E, an erythropoiesis factor, a highly glycosylated erythropoiesis factor, an angiopoietin, a hemoglobin, a thrombin, a thrombin receptor activating peptide, a thrombomodulin, blood factors VII, VIIa, VIII, IX and XIII, a plasminogen activating factor, a fibrin-binding peptide, an urokinase, a streptokinase, a hirudine, a protein C,C-reactive protein, a renin inhibitor, a collagenase inhibitor, a superoxide dismutase, a platelet-derived growth factor, an epithelial cell growth factor, an epidermal growth factor, an angiostatin, an angiotensin, a bone growth factor, a bone stimulating protein, a calcitonin, an atriopeptin, a cartilage inducing factor, an elcatonin, a connective tissue activating factor, a tissue factor pathway inhibitor, a follicle stimulating hormone, a luteinizing hormone, a luteinizing hormone releasing hormone, a nerve growth factor, a parathyroid hormone, a relaxin, a secretin, a somatomedin, an insulin-like growth factor, an adrenocortical hormone, a glucagon, a cholecystokinin, a pancreatic polypeptide, a gastrin-releasing peptide, a cortincotropin releasing factor, a thyroid stimulating hormone, an autotaxin, a lactoferrin, a myostatin, a receptor, a receptor antagonist, a cell surface antigen, a virus-derived vaccine antigen, a monoclonal antibody, a polyclonal antibody, and an antibody fragment.

In another specific embodiment of the invention, the physiologically active protein or peptide is characterized in that it is selected from the group consisting of an exendin-4, an exendin-4 derivative, a GLP-1 agonist, an insulin and a GLP-1/glucagon dual agonist.

In another specific embodiment of the invention, the exendin-4 derivative is characterized in that it is an exendin-4 derivative in which the charge on the N-terminal of exendin-4 is modified, which is selected from the group consisting of an exendin-4 derivative in which N-terminal amine group of exendin-4 is deleted, an exendin-4 derivative in which N-terminal amine group of exendin-4 is substituted with hydroxl group, an exendin-4 derivative in which N-terminal amine group of exendin-4 is substituted with carboxly group, an exendin-4 derivative in which N-terminal amine group of exendin-4 is modified with dimethyl group, and an exendin-4 derivative in which alpha carbon of N-terminal histidine residue of exendin-4 is deleted.

In another specific embodiment of the invention, the above-described internal residue is characterized in that it is a lysine residue at position 12 or 27 of the exendin-4 derivative in which N-terminal charge of exendin-4 is modified.

In another specific embodiment of the invention, the above-described internal residue is characterized in that it is a lysine residue at position 27 of the exendin-4 derivative in which N-terminal charge of exendin-4 is modified.

In another specific embodiment of the invention, the exendin-4 derivative in which the charge on the N-terminal of the exendin-4 is changed is characterized in that it is an exendin-4 derivative in which alpha carbon of N-terminal histidine residue of exendin-4 is deleted.

Another aspect, the preset the present invention provides a composition, comprising a conjugate of a physiologically active protein or peptide in which a carrier is bound to the non-terminal, internal residue of a physiologically active protein or peptide, via a non-peptidyl linker, wherein the conjugate exhibits decreased immunogenicity as compared to that of the physiologically active protein or peptide to which the carrier is not bound.

In one specific embodiment of the invention, the above-described conjugate is characterized in that it has decreased immunogenicity, which is a side effect of a long-acting preparation.

In another specific embodiment of the invention, the non-peptidyl linker is characterized in that it is a polyethylene glycol.

Another aspect, the present invention provides a method for preparing the conjugate of the physiologically active protein or peptide, in which the carrier is bound to the non-terminal, internal residue of the physiologically active protein or peptide.

### Advantageous Effects of Invention

The physiologically active protein or peptide conjugate of the present invention can significantly decrease immunogenicity in the human body and thus reduce antibody production rate against proteins or peptides. Therefore, the present conjugate has advantages in that the phenomenon of reduced clinical effects of the physiologically active protein or peptide is low, and it can be effectively used in the development of long-acting formulations having a high safety against the immune response.

### Brief Description of Drawings

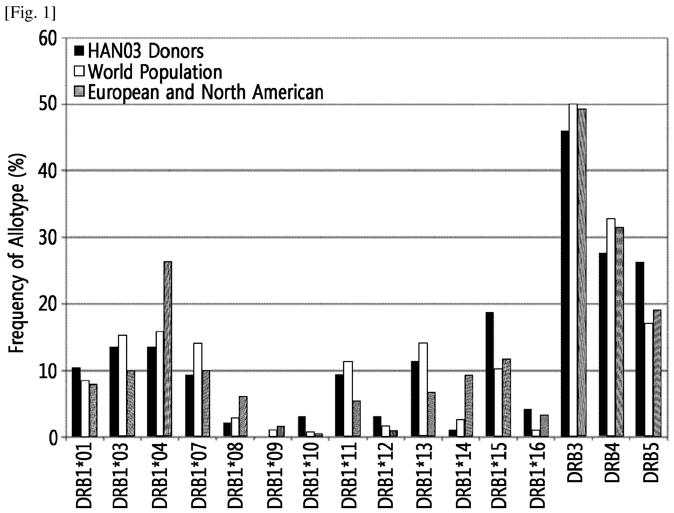

Figure 1 is a diagram showing a comparison of HLA-DR genotype frequency of a donor in the *ex* vivo T cell activity test with that of the population in the world, Europe and North America.

### Best Mode for Carrying out the Invention

The present invention relates to a method for decreasing the immunogenicity of a physiologically active protein or peptide compared to that of the protein or peptide to which a carrier has not been bound, which comprises a step of binding a carrier to the non-terminal, internal residue of the physiologically active protein or peptide.

In the present invention, the inventors have discovered a method for decreasing the immunogenicity of a physiologically active protein or peptide in which a non-peptide linker and Fc fragment are bound to the internal residue rather than the terminal of a physiologically active protein or peptide, thus inhibiting the mechanism in which the desired protein or peptide acts as an antigen. The inventors have identified that, in the case of using the method as described above, the activation of T cells and the antibody production reaction in animals is significantly inhibited compared with the method for preparing a conjugate by the modification at other sites such as N-terminal of the peptide. As a result, the present inventors have found that the physiologically active protein or peptide conjugate used as a conventional protein pharmaceutical preparation has a novel use as the composition and method for deacreasing the immunogenicity of a physiologically active protein or peptide.

The decrease of immunogenicity in the body can be measured without limitation by a known method. For example, the difference in immunogenicity can be confirmed by the ex-vivo activity measurement method of T cells which comprises coupling each of the carriers to the N-terminal or the sites other than the N-terminal including the C-terminal. Aldehyde reactive group selectively reacts with the N-terminal at a low pH, and also it can form a covalent bond with a lysine residue at the condition of high pH, for example pH 9.0. The pegylation reaction is conducted while changing the reaction pH, and then positional isomers can be separated from the reaction mixture using an ion exchange column.

When the coupling is made at a position other than N-terminal end which is an important site in the activity of the protein or peptide *in* vivo, a reactive thiol group can be introduced to an amino acid residue position to be modified, thus forming a covalent bond between the protein or peptide and a maleimide group of the non-peptidyl polymer.

When the coupling is made at a position other than N-terminal end which is an important site in the activity of the protein or peptide *in* vivo, an amine group is introduced to an amino acid residue position to be modified, thus forming a covalent bond between the protein or peptide and an aldehyde group of the non-peptidyl polymer.

The method of protection of the N-terminal end includes methylation, deamination or acetylation in addition to dimethylation, but is not limited thereto.

In the present invention, "physiologically active protein or peptide" refers to a protein or peptide that can control the genetic expression or physiological function. The physiologically active protein or peptide can be included, without limitation, in the scope of the present invention, as long as a carrier is bound to the non-terminal, internal residue of the physiologically active protein or peptide according to the present invention, thus exhibiting descresed immunogenicity compared to that of the protein or peptide to which a carrier is not bound. As described below, the carrier can be bound via a linker, specifically a non-peptidyl linker, to a physiologically active protein or peptide.

In addition, the physiologically active protein or peptide includes, in addition to native biologically active protein or peptide, derivatives, variants, or fragments thereof.

Examples of the physiologically active protein or peptide include an anti-obesity peptide, an insulinotropic peptide or an analog thereof, a leptin, an insulin, an insulin analog, a glucagon, a human growth hormone, a growth hormone releasing hormone, a growth hormone releasing peptide, an interferon, an interferon receptor, a colony stimulating factor, a glucagon-like peptide (GLP-1, etc.), a GLP-1/glucagon dual agonist, a gastric inhibitory polypeptide (GIP), a G-protein-coupled receptor, an interleukin, an interleukin receptor, an enzyme, an interleukin binding protein, a cytokine binding protein, a macrophage activating factor, a macrophage peptide, a B cell factor, a T cell factor, a protein A, an allergy inhibitory factor, a cell necrosis glycoprotein, an immunotoxin, a lymphotoxin, a tumor necrosis factor, a tumor inhibitory factor, a metastasis growth factor, an alpha-1 antitrypsin, an albumin, an α-lactalbumin, an apolipoprotein-E, an erythropoiesis factor, a highly glycosylated erythropoiesis factor, an angiopoietin, a hemoglobin, a thrombin, a thrombin receptor activating peptide, a thrombomodulin, blood factors VII, VIIa, VIII, IX and XIII, a plasminogen activating factor, a fibrin-binding peptide, an urokinase, a streptokinase, a hirudine, a protein C, C-reactive protein, a renin inhibitor, a collagenase inhibitor, a superoxide dismutase, a platelet-derived growth factor, an epithelial cell growth factor, an epidermal cell growth factor, an angiostatin, an angiotensin, a bone growth factor, a bone stimulating protein, a calcitonin, an atripeptin, a cartilage inducing factor, an elcatonin, a connective tissue activating factor, a tissue factor pathway inhibitor, a follicle stimulating hormone, a luteinizing hormone, a luteinizing hormone releasing hormone, a nerve growth factor, a parathyroid hormone, a relaxin, a secretin, a somatomedin, an insulin-like growth factor, an adrenocortical hormone, a glucagon, a cholecystokinin, a pancreatic polypeptide, a gastrin-releasing peptide, a cortincotropin releasing factor, a thyroid stimulating hormone, an autotaxin, a lactoferrin, a myostatin, a receptor, a receptor antagonist, a cell surface antigen, a virus-derived vaccine antigen, a monoclonal antibody, a polyclonal antibody, and an antibody fragment, without limitation.

More specifically, the physiologically active protein or peptide may include an insulin, an insulinotropic peptide, or a GLP-1/glucagon dual agonist, but is not limited thereto.

In the present invention, the term "insulin" includes all peptides or modified peptides which have a stimulating effect on insulin receptors. The insulin may be, for example, a native insulin, a rapid-acting insulin, a basal insulin, an insulin analog in which any amino acids of the native insulin is changed by any one method selected from substitution, addition, deletion, and modification, or a combination of these methods, or may be a fragment thereof. Also, the insulin used in the present invention may be a long-acting insulin to which long-acting techniques applied to overcome the short half-life. In particular, the insulin may be a long-acting insulin or a long-acting insulin analog which can be administered once a week, but is not limited thereto.

Some specific examples of the insulin according to the present invention include an insulin or an insulin analog and its long-acting type as disclosed in Korean Patent No. 10-1058290 (or International Publication WO 2008-082274) or Korean Patent Application Publication No. 2014-0106452 (or International Publication WO 2014-133324), the entire contents of which are incorporated herein by reference, but are not limited thereto.

As used herein, the term "insulin analog" refers to a substance which retains the same function of controlling the blood glucose level *in* vivo as a native insulin. Specifically, the insulin analogs include those in which one or more amino acids in the native insulin sequence have been modified. The insulin analog may be an insulin analog in which A-chain or B-chain amino acid of native insulin is changed. The native insulin amino acid sequence is as follows.
A chain: 
Gly-Ile-Val-Glu-Gln-Cys-Cys-Thr-Ser-Ile-Cys-Ser-Leu-Tyr-Gln-Leu-Glu-Asn-Tyr-
Cys-Asn (SEQ ID NO: 1)
B chain:

Phe-Val-Asn-Gln-His-Leu-Cys-Gly-Ser-His-Leu-Val-Glu-Ala-Leu-Tyr-Leu-Val-Cys
-Gly-Glu-Arg-Gly-Phe-Phe-Tyr-Thr-Pro-Lys-Thr (SEQ ID NO: 2)

Specifically, at least one amino acid in the native insulin may have a modififation selected from the group consisting of substitution, addition, deletion, modification and a combination thereof, but are not limited thereto.

In the substitution or addition of the amino acids, 20 amino acids that are normally observed in a human protein as well as atyhpical or non-naturally occurring amino acids can be used. The commercial sources of the atypical amino acids may include Sigma-Aldrich, ChemPep and Genzyme pharmaceuticals. The peptides including such amino acids and a typical peptide sequence can be synthesized or purchased from commercial peptide synthesis companies, for example, American peptide company Inc., and Bachem (USA), or Anygen (Korea).

Specifically, the above-described insulin analogs include an inverted insulin, an insulin variant, an insulin fragment, an insulin agonist, an insulin derivative and the like, and the preparation method thereof includes a genetic recombination as well as a solid phase method, but is not limited thereto.

The term "insulin derivative" shows an amino acid sequence homolgy with A-chain and B-chain of native insulin, while retaining the function to control the blood glucose level in the body, and includes a peptide form which may have some groups on the amino acid redidues chemically substituted (e.g., alpha-methylation, alpha-hydroxylation), deleted (e.g., deamination), or modified (e.g., N-methylation). In addition, the insulin derivative includes a peptide mimic, and a low molecular or high molecular compound, which can bind with an insulin receptor to control blood glucose levels in the body, even without homology with a native insulin and an amino acid sequence.

As used herein, the term "insulin fragment" refers to a fragment having one or more amino acids added or deleted in insulin. The added amino acid may be an amino acid that is not present in the native state (e.g., D-type amino acid). Such insulin fragment retains a function to control blood glucose levels in the body.

As used herein, the term "insulin variant" is a peptide having one or more amino acid sequences different from those of insulin, and retaining a function to control blood glucose levels in the body.

Methods for preparing the insulin agonist, derivative, fragment and variant of the present invention, respectively, can be used alone and in combination thereof. For example, the present invention includes a peptide which has one or more amino acid sequence different from those of native insulin, has deamination at the terminal amino acid residue, and retains a function to control blood glucose levels in the body, can be included.

The description of the agonists, derivatives, fragments and variants may be applied evewn to other types of proteins or peptides.

Specifically, the insulin analogs may be those in which one or more amino acids selected from the group consisting of amino acids at position 1, amino acids at position 2, amino acids at position 3, amino acids at position 5, amino acids at position 8, amino acids at position 10, amino acids at position 12, amino acids at position 16, amino acids at position 23, amino acids at position 24, amino acids at position 25, amino acids at position 26, amino acids at position 27, amino acids at position 28, amino acids at position 29, amino acids at position 30 of the chain B; amino acids at position 1, amino acids at position 2, amino acids at position 5, amino acids at position 8, amino acids at position 10, amino acids at position 12, amino acids at position 14, amino acids at position 16, amino acids at position 17, amino acids at position 18, amino acids at position 19 and amino acids at position 21 of the chain A have been substituted with other amino acids, and more specifically those in which one or more amino acids selected from the group consisting of amino acids at position 8, amino acids at position 23, amino acids at position 24, amino acids at position 25 of the chain B; amino acids at position 1, amino acids at position 2, amino acids at position 14 and amino acids at position 19 of the chain A have been substituted with other amino acids.

Specifically, among the foregoing amino acids, those in which one or more, two or more, three or more, four or more, five or more, six or more, seven or more, eight or more, nine or more, ten or more, 11 or more, 12 or more, 13 or more, 14 or more, more than 15, 16 or more, 17 or more, 18 or more, 19 or more, 20 or more, 21 or more, 22 or more, 23 or more, 24 or more, 25 or more, 26 or more, or 27 or more amino acids have been substituted with other amino acids may be used, but are not limited thereto.

The amino acid residues at the above-described positions may be substituted with alanine, glutamic acid, asparagine, isoleucine, valine, glutamine, glycine, lysine, histidine, cysteine, phenylalanine, tryptophan, proline, serine, threonine, and/or aspartic acids.

In the present invention, "insulinotropic peptide" refers to a peptide that retains the function of secreting insulin. The insulinotropic peptide may stimulate synthesis or expression of insulin in the beta cells of the pancreas. Specifically, the insulinotropic peptide is GLP (Glucagon like peptide)-1, exendin-3, or exendin-4, but is not limited thereto. The insulinotropic peptide includes native insulinotropic peptides, precursors thereof, agonists thereof, derivatives thereof, fragments thereof, and variants thereof. Further, a combination thereof as previously described can be included.

GLP-1 is a hormone secreted by the small intestine, and generally promotes biosynthesis and secretion of insulin, inhibits glucagon secretion, and promotes glucose uptake by cells. In the small intestine, a glucagon precursor is decomposed into three peptides, that is, glucagon, GLP-1, and GLP-2. Here, the GLP-1 means GLP-1 (1-37), which is originally in the form having no insulinotropic function, but is then processed and converted into the activated GLP-1 (7-37) forms.

Exendin-4 refers to peptides having 39 amino acids, which show a 53% amino acid sequence homology with GLP-1. The exendin-4 may have the following sequence, but is not limited thereto:
Exendin-4:

His Gly Glu Gly Thr Phe Thr Ser Asp Leu Ser Lys Gln Met Glu Glu Glu Ala Val
Arg Leu Phe Ile Glu Trp Leu Lys Asn Gly Gly Pro Ser Ser Gly Ala Pro Pro Pro Ser
(SEQ ID NO: 3)

Meanwhile, exendin-3 is a polypeptide having different amino acids at positions 2 and 3 from those of exendin-4. Exendin-3 is that in which amino acids at positions 2 and 3 of exendin 4 are substituted with serine and aspartic acid, respectively, and it can be represented as Ser²Asp³-exendin-4(1-39). Specifically, the exendin-3 may have the following sequence, but is not limited thereto:
Exendin-3:

His Ser Asp Gly Thr Phe Thr Ser Asp Leu Ser Lys Gln Met Glu Glu Glu Ala Val
Arg Leu Phe Ile Glu Trp Leu Lys Asn Gly Gly Pro Ser Ser Gly Ala Pro Pro Pro Ser
(SEQ ID NO: 4)

The above-described insulinotropic peptide derivative may be that in which N-terminus of the insulinotropic peptide has been modified. More specifically, the insulinotropic peptide derivative can cause a rapid dissociation of the receptor by changing the charge on the N-terminal, and it may be a derivative in which the positive charge on the N-terminal is changed to neutral or net negative charges.

The insulinotropic peptide derivative of the present invention may include a desamino-histidyl derivative where the N-terminal amino (or amine) group of insulinotropic peptide is deleted, beta-hydroxy imidazopropionyl-derivative where the amino group is substituted with a hydroxyl group, dimethyl-histidyl derivative where the amino group is modified with two methyl groups, beta-car-boxyimidazopropionyl-derivative where the N-terminal amino group is substituted with a carboxyl group, or an imidazoacetyl-derivative where the alpha carbon of the N-terminal histidine residue is deleted to retain only the imidazoacetyl group and thus the positive charge of the amino group is removed, and other N-terminal amino group-modified derivatives are included within the scope of the present invention.

By way of example, the insulinotropic peptide derivative may be a derivative in which N-terminal amino (or amine) group or amino acid residue of exendin-4 is chemically modified. Specifically, it is an exendin-4 derivative which is prepared by substituting or removing the alpha amino group present in the alpha carbon of the N-terminal histidine residue (the first amino acid) of exendin-4. More specifically, it can include desamino-histidyl-exendin-4 (DA-Exendin-4) with removal of the N-terminal amino group, beta-hydroxy imidazopropyl-exendin-4 (HY-exendin-4) prepared by substitution of the N-terminal amino group with a hydroxyl group, beta-carboxy imidazopropyl-exendin-4 (CX-exendin-4) prepared by substitution of the N-terminal amino group with a carboxyl group, dimethyl-histidyl-exendin-4 (DM-exendin-4) prepared by modification of the N-terminal amino group with two methyl residues, or imidazoacetyl-exendin-4 (CA-exendin-4) with removal of alpha carbon of N-terminal histidine residue, and the like.

It is obvious that the insulinotropic peptide as disclosed in Korean Patent Application Publication No. 10-2012-0135123 (or international publication WO 2012/165915) or international publication WO 2014/107035 is also included in the scope of the present invention. The entire contents of these publications are incorporated herein by reference.

In the present invention, the "GLP-1/glucagon dual agonist" includes peptides or fragments, precursors, variants or derivatives thereof which have GLP-1/glucagon dual activity, like oxyntomodulin, a native GLP-1/glucagon dual agonist. In the present invention, the GLP-1/glucagon dual agonist may be a GLP-1/glucagon dual agonist to which the long-acting techniques applied to overcome the short half-life, and preferably a long-acting GLP-1/glucagon dual agonist which can be administered once a week, but is not limited thereto.

The GLP-1/glucagon dual agonist includes oxyntomodulin.

The "oxyntomodulin" refers to a peptide produced from a pre-glucagon, a percursor of glucagon. In the present invention, oxyntomodulin includes a native oxyntomodulin, a precursor thereof, a derivative thereof, a fragment thereof, a variant thereof and the like as previously described.

The oxyntomodulin may have specifically the amino acid sequence of HSQGTFTS-DYSKYLDSRRAQDFVQWLMNTKRNRNNIA (SEQ ID NO: 5), but is not limited thereto.

The oxyntomodulin derivative includes a peptide, a peptide derivative or a peptide mimic that is prepared by the addition, deletion or substitution of any amino acid of sequences of oxyntomodulin and can activate both GLP-1 receptor and glucagon receptor, and particularly, can activate each receptor at a higher level compared to the level activated by native oxyntomodulin.

Some specific examples of the GLP-1/glucagon dual agonist according to the present invention include a GLP-1/glucagon dual agonist and its derivative or its long-acting type as disclosed in Korean Patent Application Publication Nos. 10-20125-01372771 (or International Publication WO 2012-169798) and 10-2012-01639579 (or International Publication WO 2012-173422), the entire contents of which are incorporated herein by reference.

In the present invention, the carrier that is bound to the physiological active protein or peptide may be a material which can increase the *in* vivo half-life of the physiological active protein or peptide.

Examples of the physiologically active protein or peptide include various substances capable of reducing the renal clearance of the physiologically active protein or peptide, for example, a polyethylene glycol, a fatty acid, a cholesterol, an albumin or a fragment thereof, an albumin-binding substance, a polymer of repeating units of a particular amino acid sequence, an antibody, an antibody fragment, a FcRn binding substance, an *in*-vivo connective tissue or a derivative thereof, a nucleotide, a fibronectin, a transferrin, an elastin-like polypeptide(ELP), a XTEN polypeptide, a carboxy-terminal peptide (CTP), a structure inducing probe (SIP), a saccharide, a high molecular polymer, a particular amino acid sequence, a polymer of repeating units of a particular amino acid sequence, and the like. In addition, the linkage between the physiologically active protein or peptide and the carrier includes a genetic recombination and an *in vitro* linkage, but is not limited thereto.

The carrier may be covalently or non-covalently linked to the physiologically active protein or peptide. The above described FcRn binding substance may be an immunoglobulin Fc region, for example, IgG Fc.

When polyethylene glycol is used as the carrier, a Recode technique by Ambrx Inc. which enables a site-specific binding to polyethylene glycol may be used. Also, a gly-copegylation technique by Neose company which enables a specific binding to the glycosylated moiety may be used. Furthermore, a releasable PEG technique in which polyethylene glycol is slowly deleted in the body may be used, but is not limited thereto. Also, the techniques which can be used in the present invention include techniques which increase bioavailability using PEG. In addition, the non-peptidyl polymers such as polyethylene glycol, polypropylene glycol, ethylene glycol-propylene glycol copolymer, polyoxyethylated polyol, polyvinyl alcohol, polysaccharides, dextran, polyvinyl ethyl ether, biodegradable polymer, lipid polymer, chitins, or hyaluronic acid can also be bound to the physiologically active protein or peptide using the above described techniques.

When albumin is used as a carrier, the technique which can be used in the present invention includes a technique in which albumins or albumin fragments can be directly covalently linked to the physiologically active protein or peptide to increase the *in* vivo stability. Even if albumin is not directly linked, a technique in which the albumin binding materials, for example, albumin-specific binding antibody or antibody fragment are bound to the physiologically active protein or peptide to thereby bind to albumin can be used, and a technique in which a certain peptide/protein having a binding affinity to albumin is bound to the physiologically active protein or peptide can be used. In addition, a technique in which a fatty acid having a binding affinity to albumin is bound to the physiologically active protein or peptide can be used, but is not limited thereto. Any technique or binding method which can increase the *in* vivo stability using albumin can be included here.

The technique for binding to the physiologically active protein or peptide by using the antibody or antibody fragment as a carrier in order to increase the *in* vivo half-life may also be included in the present invention. The antibody or antibody fragment having a FcRn binding site can be used, and any antibody fragment containing no FcRn binding site such as Fab can be used. CovX-body technique of CovX company using a catalytic antibody can be included herein, and the technique which increases the *in* vivo half-life using the immunoglobulin Fc region may be included in the present invention.

When the immunoglobulin Fc region is used, the linker binding to the Fc region and the physiologically active protein or peptide and its binding method may include a peptide bond or a polyethylene glycol or the like, but is not limited thereto and any chemical binding method may be available. In addition, the binding ratio of the Fc region and the insulin analog may be 1:1 or 1:2, but is not limited thereto.

An immunoglobulin constant region including Fc region is a biodegradable polypeptide which can be metabolized *in* vivo, so that it can safely be used as a drug carrier. In addition, an immunoglobulin Fc region is more advantageous in terms of production, purification and production yield of a complex than an entire immunoglobulin molecule owing to its relatively lower molecular weight. Further, since it is devoid of Fab, which exhibits high non-homogeneity due to the difference in amino acid sequence from one antibody to another, the immunoglobulin Fc alone provides the complex with significantly enhanced homogeneity, and reduces the possibility of inducing blood antigenicity.

Also, the aforementioned PEG is non-specifically bound to a specific site or various sites of the target peptide and thus increases the molecular weight of the peptide. Therefore, the PEG is effective in inhibiting the renal clearance and preventing hydrolysis and further it does not cause special side effects. In addition, when PEG is bound to an exogenous peptide, it can inhibit the recognition of antigenic sites being present in the exogenous peptide by the immune cells. Specifically, the PEG can inhibit the peptide to be phagocytosed by antigen presenting cell and proteolysed. Therefore, it is able to lower the potential for the peptide to act as an antigen. Especially for the exogenous protein to stimulate the activation of CD4+T cells as an antigen, about 14-24 short peptides in the form of being bound to MHC class II must be presented on the antigen-presenting cells. This can be inhibited in the course of being degraded as an appropriate size depending on the binding site of PEG.

In one embodiment of the present invention, the carrier and the physiologically active protein or peptide is connected via a linker, in particular, a non-peptidyl linker.

In the present invention, the non-peptidyl linker refers to a biocompatible polymer including two or more repeating units, the repeating units being bound with each other by any covalent bond excluding a peptide linkage. The non-peptidyl linker may be interchangeably used with the non-peptidyl polymer.

The non-peptidyl linker useful in the present invention may be selected from the group consisting of a biodegradable polymer, a lipid polymer, a chitin, a hyaluronic acid, and a combination thereof. The biodegradable polymer used herein may be polyethylene glycol, polypropylene glycol, ethylene glycol-propylene glycol copolymer, polyoxyethylatedpolyol, polyvinyl alcohol, polysaccharide, dextran, polyvinyl ethyl ether, polylactic acid (PLA) or polylactic-glycolic acid (PLGA). In one specific embodiment of the present invention, the non-peptidyl polymer is polyethylene glycol. In addition, derivatives thereof known in the art and derivatives easily prepared by a method known in the art may be included in the scope of the present invention.

The peptide linker which is used in the fused protein obtained by a conventional inframe fusion method has drawbacks in that it is easily cleaved *in* vivo by a proteolytic enzyme, and thus a sufficient effect of increasing the serum half-life of the active drug by a carrier cannot be obtained as expected. However, since the non-peptydyl polymer of the present invention is a substance that has no peptide linkage, it can have basically a resistance to the proteolytic enzyme, thus increasing the serum half-life of the peptide. The molecular weight of the non-peptidyl polymer which can be used in the present invention ranges specifically from 1 to 100 kDa, and more specifically from 1 to 20 kDa. The non-peptidyl polymer of the present invention, linked to the immunoglobulin Fc region, may be one type of polymer or a combination of different types of polymers.

In the present invention, the carrier is characterized in that it is bound to a non-terminal internal residue of the physiologically active protein or peptide. In this case, as described above, the carrier may be bound to the non-terminal internal residue of the physiologically active protein or peptide via a linker.

The non-terminal internal residue of the physiologically active protein or peptide may include, without limitation, any residue if it can, when a carrier is bound to the physiologically active protein or peptide, decrease the immunogenicity thereof, compared to that of a protein or peptide to which a carrier is not bound or a protein or peptide in which a carrier is bound to terminal site of the protein or peptide.

The non-terminal, internal amino acid of the physiologically active protein or peptide may be lysine, cysteine, or the like.

More specifically, when the physiologically active protein or peptide is an insulinotropic peptide, particularly exendin-4 or a derivative of exendin-4, its internal residue may be lysine residues at positions 12 or 27, but is not limited thereto.

In addition, when using an aldehyde linker as the non-peptidyl polymer, the N-terminal is reacted with an amine group in the lysine residue, and a modified form of insulinotropic peptide can be used to improve the reaction yield. For example, a reactive amine group can be maintained at a desired position using a method of blocking the N-terminal, a method of substitutig the lysine residue, a method of introducing an amine group, and further the pegylation and coupling yield can be improved.

In a preferred embodiment of the present invention, an insulinotropic peptide conjugate in which a carrier is bound to the non-terminal internal resiue of the insulinotropic peptide of the invention, refers to an insulinotropic peptide conjugate in which an immunoglobulin Fc region is specifically bound with an amine group other than the N-terminal of the insulinotropic peptide.

In one specific embodiment, the present inventors have conducted a series of experiments; that is, in a method for selectively binding PEG to a lysine residue of the insulinotropic peptide, when binding PEG to a native exendin-4, the reaction was conducted at pH 9.0, thus inducing a pegylation to lysine residue; whereas in the other method, when binding PEG to a N-terminus-removed or protected form of exendin-4 derivative, the reaction was conducted at pH 7.5, thus inducing a pegylation to lysine residue. As a result, it was comfimed that, contray to the N-terminal binding, when bound to the lysine residue, the *ex* vivo T-cell antivities were significantly inhibited (Tables 2 to 4).

Further, the term "immunoglobulin Fc region" as used herein refers to the heavy-chain constant region 2 (CH2) and the heavy-chain constant region 3 (CH3) of an immunoglobulin, excluding the variable regions of the heavy and light chains, heavy-chain constant region 1 (CH1) and the light-chain constant region 1 (CL1) of the immunoglobulin. It may further include a hinge region at the heavy-chain constant region.

Also, the immunoglobulin Fc region of the present invention may contain a part or all of the Fc region including the heavy-chain constant region 1 (CH1) and/or the light-chain constant region 1 (CL1), except for the variable regions of the heavy and light chains of the immunoglobulin, as long as it has a physiological effect substantially similar to or better than that of the native protein. Furthermore, the immunoglobulin Fc region may be a fragment having a deletion in a relatively long portion of the amino acid sequence of CH2 and/or CH3. That is, the immunoglobulin Fc region of the present invention may comprise 1) a CH1 domain, a CH2 domain, a CH3 domain and a CH4 domain, 2) a CH1 domain and a CH2 domain, 3) a CH1 domain and a CH3 domain, 4) a CH2 domain and a CH3 domain, 5) a combination of one or more domains and an immunoglobulin hinge region (or a portion of the hinge region), and 6) a dimer of each domain of the heavy-chain constant regions and the light-chain constant region.

Further, the immunoglobulin Fc region of the present invention includes a native amino acid sequence as well as a sequence derivative (mutant) thereof. An amino acid sequence derivative has a different sequence due to a deletion, an insertion, a non-conservative or conservative substitution or combinations thereof of one or more amino acid residues of the native amino acid sequences. For example, in an IgG Fc, amino acid residues at positions 214 to 238, 297 to 299, 318 to 322, or 327 to 331, known to be important in the binding, may be used as a suitable target for modification. Further, various kinds of derivatives are possible, including one in which a region capable of forming a disulfide bond is deleted, or certain amino acid residues are removed at the N-terminal end of a native Fc form or a methionine residue is added thereto. Further, to remove effector functions, a deletion may occur in a complement-binding site, such as a Clq-binding site and an antibody dependent cell mediated cytotoxicity (ADCC) site. Techniques of preparing such sequence derivatives of the immunoglobulin Fc region are disclosed in International Publications, WO 97/34631, WO 96/32478 and the like.

Amino acid exchanges in proteins and peptides, which do not wholly alter the activity of the moleculars, are known in the art (H. Neurath, R. L. Hill, The Proteins, Academic Press, New York, 1979). The most commonly occurring exchanges are exchanges between amino acid residues AlalSer, Val/Ile, Asp/Glu, Thr/Ser, AlalGly, AlalThr, Ser/Asn, AlalVal, Ser/Gly, Thy/Phe, AlalPro, Lys/Arg, Asp/Asn, Leu/Ile, Leu/Val, AlalGlu and Asp/Gly.

In addition, the Fc region, if desired, may be modified by phosphorylation, sulfation, acrylation, glycosylation, methylation, farnesylation, acetylation, amidation, and the like.

The above-described Fc derivatives may be derivatives that exhibit the same biological activity as the Fc region of the present invention or improve a structural stability against heat, pH or the like of the Fc region.

Furthermore, these Fc regions may be obtained from native forms isolated from humans and other animals including cows, goats, pigs, mice, rabbits, hamsters, rats or guinea pigs, or may be recombinants or derivatives thereof, obtained from transformed animal cells or microorganisms. Herein, the method for obtaining from a native immunoglobulin includes isolating whole immunoglobulins from human or animal organisms and then treating them with a proteolytic enzyme. Papain treatment results in the digestion of the native immunoglobulin into Fab and Fc, and pepsin treatment results in the production of pFc' and F(ab)2 fragments. These fragments may be subjected to size exclusion chromatography and the like to isolate Fc or pFc' fragments.

Specifically, a human-derived Fc region is a recombinant immunoglobulin Fc region that is obtained from a microorganism.

In addition, the immunoglobulin Fc region be in the form of having native sugar chains, increased sugar chains compared to a native form or decreased sugar chains compared to the native form, or may be in a deglycosylated form. The increase, decrease or removal of the immunoglobulin Fc sugar chains may be achieved by methods common in the art, such as a chemical method, an enzymatic method and a genetic engineering method using a microorganism. The removal of sugar chains from an immunoglobulin Fc region results in a sharp decrease in binding affinity to the C1q part of the complement component and a decrease or removal in antibody-dependent cell-mediated cytotoxicity or complement-dependent cytotoxicity, thereby not inducing unnecessary immune responses *in* vivo. In this regard, an immunoglobulin Fc region in a deglycosylated or aglycosylated form may be more suitable to the object of the present invention as a drug carrier.

As used herein, the term "deglycosylation" refers to enzymatically removing sugar moieties from an Fc region, and the term "aglycosylation" means that an Fc region is produced in an unglycosylated form by a prokaryote, specifically *E*. coli.

Meanwhile, the immunoglobulin Fc region may be derived from humans or other animals including cows, goats, pigs, mice, rabbits, hamsters, rats and guinea pigs, and preferably from humans.

Also, the immunoglobulin Fc region may be an Fc region that is derived from IgG, IgA, IgD, IgE and IgM, or that is made by combinations thereof or hybrids thereof. Specifically, it is derived from IgG or IgM, which are among the most abundant proteins in human blood, and most specifically from IgG, which is known to enhance the half-lives of ligand-binding proteins, but is not limited thereto.

On the other hand, the term "combination", as used herein, means that polypeptides encoding single-chain immunoglobulin Fc regions of the same origin are bound to a single-chain polypeptide of a different origin to form a dimer or multimer. That is, a dimer or multimer may be formed from two or more fragments selected from the group consisting of IgG Fc, IgA Fc, IgM Fc, IgD Fc, and IgE Fc fragments.

In the present invention, the term "hybrid" means that a sequence corresponding to at least two Fc fragments of a different origin is present in a single-chain immunoglobulin Fc region. In the present invention, various types of hybrid are available. That is, the hybrid consisting of 1 to 4 domains selected from the group consisting of CH1, CH2, CH3 and CH4 of IgG Fc, IgM Fc, IgA Fc, IgE Fc and IgD Fc is available, and may include a hinge. On the other hand, IgG can also be divided into sub-classes of IgG1, IgG2, IgG3 and IgG4, and in the present invention, a combination or hybridization thereof is possible. It is specifically sub-classes of IgG2 and IgG4, and most specifically Fc region of IgG4 rarely having effector function, such as a complement dependent cytotoxicity (CDC).

That is, the immunoglobulin Fc region for the carrier of the drug of the present invention may be, for example, human IgG4-derived aglycosylated Fc region, but is not limited thereto. The human-derived Fc region is preferable as compared with nonhuman-derived Fc region which can cause undesirable immune responses, for example, which can act as an antigen in the human body to produce a new antibody.

The non-peptidyl polymer used in one specific embodiment of the present invention has a reactive group capable of binding to the immunoglobulin Fc region and the physiologically active protein or peptide. In a further specific embodiment, this reactive group is located at both terminal ends. The both terminal reactive group of the non-peptidyl polymer is preferably selected from the group consisting of a reactive aldehyde group, a propionaldehyde group, a butyraldehyde group, a maleimide group and a succinimide derivative. The succinimide derivative may be succinimidyl propionate, hydroxy succinimidyl, succinimidyl carboxymethyl, or succinimidyl carbonate. In particular, when the non-peptidyl polymer has a reactive group of the reactive aldehyde group at both terminal ends thereof, it is effective in linking at both terminal ends with a physiologically active polypeptide and an immunoglobulin with minimal non-specific reactions. A final product produced by reductive alkylation by an aldehyde linkate is much more stable than that bound by an amide linkage. The aldehyde reactive group selectively reacts at an N-terminus at a low pH, and forms a covalent bond with a lysine residue at a high pH, such as pH 9.0.

The both terminal reactive groups of the non-peptidyl polymer may be the same as or different from each other.

For example, the non-peptidyl polymer may possess a maleimide group at one terminal end, and an aldehyde group, a propionaldehyde group or a butyraldehyde group at the other terminal end. When a polyethylene glycol having a reactive hydroxy group at both terminal ends thereof is used as the non-peptidyl polymer, the hydroxy group may be activated to various reactive groups by known chemical reactions, or a polyethylene glycol having a commercially available modified reactive group may be used to thereby prepare a physiologically active protein or peptide conjugate, specifically an insulinotropic peptide conjugatge, according to the present invention.

The insulinotropic peptide conjugate of the present invention can not only maintain *in* vivo activities of a conventional insulinotropic peptide, such as a promotion of insulin synthesis and secretion, an appetite suppression, a weight loss, an increase in blood glucose sensitivity of beta cells, a promotion of beta cell proliferation, or a gastric emptying delay, but also it can dramatically increase the serum half-life of the insulinotropic peptide and hence *in* vivo lasting effects of the peptide. Accordinlgy, this insulinotropic peptide conjugate is useful in the treatment of diabetes, obesity, acute coronary syndrome or polycystic ovary syndrome.

In another embodiment, the present invention provides a composition, comprising a conjugate of a physiologically active protein or peptide in which a carrier is bound to the non-terminal, internal residue of a physiologically active protein or peptide, via a non-peptidyl linker, wherein the conjugate exhibits decreased immunogenicity as compared to that of the physiologically active protein or peptide to which the carrier is not bound.

Specifically, the above-described conjugate is characterized in that it decreases immunogenicity, which is a side effect of a long-acting preparation.

Moreover, the non-peptidyl linker may be polyethylene glycol.

The physiologically active protein or peptide, the linker and the conjucate are as described above.

In another aspect, the present invention provides a method for preparing the conjugate of the physiologically active protein or peptide.

In detail, the present invention provides a method for preparing the conjugate of the physiologically active protein or peptide which comprises the follwing steps:
(1) covalently binding a non-peptidyl polymer having aldehyde, maleimide or succinimid reactive groups at the both terminal ends to an amine or thiol group of the physiologically active protein or peptide;
(2) separating the physiologically active protein or peptide which is covalently bound to the non-peptidyl polymer through a site other than the N-terminal end of the physiologically protein or peptide from the reaction mixture of step (1); and
(3) covalently binding an immunoglobulin Fc region to the other terminal end of the non-peptidyl polymer covalently bound to the physiologically active protein or peptide to produce a conjugate of the physiologically active protein or peptide in which both terminal ends of the non-peptidyl polymer are bound with the immunoglobulin Fc region and the physiologically active protein or peptide, respectively.

In a preferred aspect, the present invention provides a method for preparing a protein conjugate which comprises the follwing steps:
(1) covalently binding a non-peptidyl polymer haivng aldehyde reactive groups at the both terminal ends to a lysine residue of the physiologically active protein or peptide;
(2) separating the physiologically active protein or peptide covalently bound to the non-peptidyl polymer through the lysine residue of the physiologically active protein or peptide from the reaction mixture of step (1); and
(3) covalently linking an immunoglobulin Fc region to the other terminal end of the non-peptidyl polymer covalently bound to the physiologically active protein or peptide to produce a conjugate in which both terminal ends of the non-peptidyl polymer are bound with the immunoglobulin Fc region and the physiologically active protein or peptide, respectively.

More specifically, the non-peptidyl polymer of step (1) and the lysine residue of the insulinotropic peptide, which is a physiologically active protein or peptide, are bound at pH 7.5 or higher.

### Mode for the Invention

Hereinafter, the present invention will be described in more detail by the following examples. However, the following examples are intended to illustrate the invention and not to limit the scope of the invention thereto.

### Example 1: Pegylation of exendin-4 and separation of positional isomer of pegylated exendin-4

For PEGylation of the N-terminus of native exendin-4 (American Peptides) with 3.4K PropionALD (2) PEG (PEG with two propionaldehyde groups of molecular weight of 3.4 kDa, IDB Inc., Korea), the peptide and PEG were reacted at a molar ratio of 1:15 with a peptide concentration of 3mg/ml at 4°C for 90 minutes. At this time, the reaction was conducted in a 100mM NaOAc buffer (pH 4.0), and a reducing agent, 20mM SCB (NaCNBH₃) was added thereto.

Also, for PEGylation of the lysine (Lys) of exendin-4 with 3.4K PropionALD (2) PEG, the peptide and PEG were reacted at a molar ratio of 1:30 with a peptide concentration of 3mg /ml at 4°C for 3 hours. At this time, the reaction was conducted in a 100mM Na-phosphate buffer (pH 9.0), and a reducing agent, 20mM SCB was added thereto. The mono-pegylated peptide was primarily purified from the reaction solution through a SOURCE Q (XK 16ml, Amersham Biosciences), and the isomer was separated through a SOURCES (XK 16ml, Amersham Biosciences). It could be seen that the N-terminus-pegylated peak appeared earlier, and then two lysine (Lys)-pegylated peaks appeared in order. The pegylated sites were confirmed from the eluted peak by a peptide mapping method.

The Lys12-pegylated conjugate was eluted first, and then the Lys27-pegylated conjugate was eluted in the last portion. A perfect peak separation between N-terminal positional isomer and the Lys12 positional isomer was possible.
Column : SOURCE Q (XK 16ml, Amersham Biosciences) 58-27
Flow rate : 2.0ml/min
Gradient: A 0 → 40% 80min B (A: 20mM tris pH8.5, B: A + 0.5M NaCl)
Column : SOURCE S (XK 16ml, Amersham Biosciences)
Flow rate : 2.0ml/min
Gradient: A 0 → 100% 50 min B (A : 20mM citric acid pH 3.0, B : A + 0.5M KCl)

### Example 2: Pegylation of CA exendin-4 lysine residue and separation of positional isomer

For PEGylation of the lysine (Lys) residue of CA exendin-4 (American American Peptides) with 3.4K PropionALD (2) PEG, the CA exendin-4 and PEG were reacted at a molar ratio of 1:30 with a CA exendin-4 concentration of 3mg/ml at 4°C for 3 hours. CA exendin-4 is a N-terminal-modified exendin-4 in which the alpha carbon is deleted from the N-terminal histidine residue of a native exendin and the β-carbon of the side chain is directly bound to a carboxyl carbon. At this time, the reaction was conducted in a 100mM Na-phosphate buffer (pH 9.0), and a reducing agent, 20mM SCB was added thereto. The mono-peglated peptide was primarily purified from the reaction solution through a SOURCE Q (XK 16ml, Amersham Biosciences), and the isomer was separated through a SOURCES (XK 16ml, Amersham Biosciences).

It could be seen that two lysine(Lys)-pegylated peaks appeared. The pegylated sites were confirmed from the eluted peaks by a peptide mapping method.

The Lys12-pegylated conjugate was eluted first, and then the Lys27-pegylated conjugate was eluted in the last portion. A perfect peak separation between N-terminal positional isomer N-terminal positional isomer and the Lys12 positional isomer allowed was possible
Column : SOURCE Q (XK 16ml, Amersham Biosciences)
Flow rate : 2.0ml/min
Gradient: A 0 → 40% 80min B (A: 20mM tris pH8.5, B: A + 0.5M NaCl)
Column : SOURCE S (XK 16ml, Amersham Biosciences)
Flow rate : 2.0ml/min
Gradient: A 0 → 100% 50 min B (A : 20mM citric acid pH 3.0, B : A + 0.5M KCl)

### Example 3: Preparation of imidazo-acetyl exendin-4 (Lys27)-immunoglobulin Fc conjugate

3.4K PropionALD(2) PEG was reacted with the Lys of CA exendin-4 using imidazo-acetyl exendin-4 (CA exendin-4, AP, USA) in the same manner as in Example 2. The coupling reaction was then conducted using the last isomer peak (positional isomer of Lys 27), which shows a lot of reactivity and is easily distinguished from the N-terminal isomer, among the two Lys isomer peaks. The peptide and the immunoglobulin Fc were reacted at a molar ratio of 1:8, and a total protein concentration of 60 mg/mL at 4°C for 20 hours. The reaction wsa performed in a solution of 100mM K-P (pH 6.0) and a reducing agent, 20mM SCB, was added thereto. After the coupling reaction, the two step purification using 16ml of SOURCE Q and 16ml of SOURCE ISO was the same as in Example 2. The result of the reverse phase HPLC analysis showed a purity of 95.8%.

### Example 4: Separation of human peripheral blood mononuclear cells (PBMC) for the ex vivo test and selection of the donors

Human peripheral blood mononuclear cells (PBMC) were separated within 24 hours from blood collected from healthy donors. Donating blood has been supplied by UK National Blood Transfusion Service (Addenbrooke Hospital, Cambridge, UK). The peripheral blood mononuclear cells were separated from a buffy coat obtained by a density gradient centrifugation method using Lymphoprep^{™} (Axis-shield, Dundee, Scotland). Among them, CD8+T cells were removed using CD8+ RosetteSep^{™} (StemCell Technologies Inc, London, UK). The peripheral blood mononuclear cells of each donor were stored in liquid nitrogen until before use. HLA-DR haploid genotype of the cells of the donor were analyzed using HLA SSP-PCR based tissue-typing kit (Biotest, Solihull, UK). The reactivity of the T cells was tested using KLH (Keyhole Limpet Haemocyanin, Pierce (Perbio), Northumberland, UK), which is an antigen peptide derived from influenza A and Epstein Barr virus.

50 donors representing the frequency of HLA-DR type of the world's population were selected and composed of a single cohort. MHC class II haploid genotypes and the reactivity of T cells for each donor constituting the cohort is shown in Table 1 below. The frequency of the genotype of the donor was compared with the frequency of the world's population and the results are shown in Figure 1. Table 1 below shows the HLA-DR genotypes and the reactivity of T-cells on the antigenic peptides KLH for each donor.

**[Table 1]**

| **Donor No.** | **Haplotype** | **KLH** | |
|---|---|---|---|
| | | **Test 1** | **HAN03** |
| 1 | DRB1^{∗}01,DRB1^{∗}13;DRB3^{∗} | 2.25 | 18.69 |
| 2 | DRB1^{∗}07,DRB1^{∗}12;DRB3^{∗};DRB4^{∗} | 1.11 | 1.60 |
| 3 | DRB1^{∗}03,DRB1^{∗}15;DRB3^{∗};DRB5^{∗} | 2.66 | 2.87 |
| 4 | DRB1^{∗}01,DRB1^{∗}07;DRB3^{∗};DRB4^{∗} | 5.54 | 7.54 |
| 5 | DRB1^{∗}03,DRB1^{∗}16;DRB3^{∗};DRB5^{∗} | 7.02 | 3.46 |
| 6 | DRB1^{∗}01,DRB1^{∗}13;DRB3^{∗} | 4.36 | 14.17 |
| 7 | DRB1^{∗}03,DRB1^{∗}04;DRB3^{∗};DRB4^{∗} | 4.45 | 9.98 |
| 8 | DRB1^{∗}03,DRB1^{∗}13;DRB3^{∗} | 8.85 | 4.37 |
| 9 | DRB1^{∗}01,DRB1^{∗}12;DRB3^{∗} | 4.79 | 8.45 |
| 10 | DRB1^{∗}01,DRB1^{∗}13;DRB3^{∗} | 2.53 | 3.14 |
| 11 | DRB1^{∗}07,DRB1^{∗}15;DRB4^{∗};DRB5^{∗} | 3.00 | 10.29 |
| 12 | DRB1^{∗}04,DRB1^{∗}13;DRB3^{∗};DRB4^{∗} | 2.70 | 9.31 |
| 13 | DRB1^{∗}01,DRB1^{∗}12;DRB3^{∗} | 2.55 | 15.07 |
| 14 | DRB1^{∗}11,DRB1^{∗}15;DRB3^{∗};DRB5^{∗} | 0.27 | 1.55 |
| 15 | DRB1^{∗}07,DRB1^{∗}15;DRB3^{∗};DRB5^{∗} | 3.03 | 8.78 |
| 16 | DRB1^{∗}10,DRB1^{∗}13;DRB3^{∗} | 4.08 | 4.65 |
| 17 | DRB1^{∗}07,DRB1^{∗}11;DRB3^{∗};DRB4^{∗} | **1.13** | **5.80** |
| 18 | DRB1^{∗}03,DRB1^{∗}04;DRB3^{∗};DRB4^{∗} | **0.61** | **5.34** |
| 19 | DRB1^{∗}03,DRB1^{∗}13;DRB3^{∗} | 2.42 | 12.17 |
| 20 | DRB1^{∗}04,DRB1^{∗}12;DRB3^{∗};DRB4^{∗} | 2.76 | 6.51 |
| 21 | DRB1^{∗}15;DRB5^{∗} | 3.38 | 3.27 |
| 22 | DRB1^{∗}04,DRB1^{∗}15;DRB4^{∗};DRB5^{∗} | 2.11 | 3.55 |
| 23 | DRB1^{∗}04,DRB1^{∗}11;DRB3^{∗};DRB4^{∗} | 1.93 | 3.28 |
| 24 | DRB1^{∗}13,DRB1^{∗}15;DRB3^{∗};DRB5^{∗} | 8.93 | 6.66 |
| 25 | DRB1^{∗}11,DRB1^{∗}13;DRB3^{∗} | 2.02 | 2.99 |
| 26 | DRB1^{∗}04,DRB1^{∗}07;DRB4^{∗} | 2.42 | 1.97 |
| 27 | DRB1^{∗}11,DRB1^{∗}13;DRB3^{∗} | **5.55** | **1.20** |
| 28 | DRB1^{∗}04,DRB1^{∗}11;DRB3^{∗};DRB4^{∗} | 3.97 | 3.93 |
| 29 | DRB1^{∗}03,DRB1^{∗}04;DRB3^{∗};DRB4^{∗} | 2.00 | 6.76 |
| 30 | DRB1^{∗}03,DRB1^{∗}15;DRB3^{∗};DRB5^{∗} | **1.22** | **13.32** |
| 31 | DRB1^{∗}15,DRB1^{∗}16;DRB5^{∗} | 3.95 | 5.75 |
| 32 | DRB1^{∗}03,DRB1^{∗}11;DRB3^{∗} | 2.82 | 3.74 |
| 33 | DRB1^{∗}13,DRB1^{∗}15;DRB3^{∗};DRB5^{∗} | 2.43 | 1.97 |
| 34 | DRB1^{∗}04,DRB1^{∗}15;DRB4^{∗};DRB5^{∗} | 3.79 | 4.70 |
| 35 | DRB1^{∗}01,DRB1^{∗}04;DRB4^{∗} | 9.24 | 8.67 |
| 36 | DRB1^{∗}03,DRB1^{∗}04;DRB3^{∗};DRB4^{∗} | 2.21 | 3.06 |
| 37 | DRB1^{∗}10,DRB1^{∗}15;DRB5^{∗} | 12.11 | 4.03 |
| 38 | DRB1^{∗}08,DRB1^{∗}13;DRB3^{∗} | 4.85 | 3.22 |
| 39 | DRB1^{∗}04,DRB1^{∗}11;DRB3^{∗};DRB4^{∗} | 5.37 | 6.43 |
| 40 | DRB 1*01 ,DRB 1 * 16 ;DRB5 * | 3.22 | 4.15 |
| 41 | DRB 1 *08,DRB 1*15 ;DRB5 * | 2.24 | 2.92 |
| 42 | DRB1^{∗}14;DRB1^{∗}15;DRB3^{∗};DRB5^{∗} | 20.58 | 13.67 |
| 43 | DRB1^{∗}15,DRB1^{∗}16;DRB5^{∗} | 3.50 | 4.88 |
| 44 | DRB1^{∗}15;DRB5^{∗} | 2.01 | 7.01 |
| 45 | DRB1^{∗}07,DRB1^{∗}11;DRB3^{∗};DRB4^{∗} | 1.93 | 13.71 |
| 46 | DRB1^{∗}01,DRB1^{∗}04;DRB4^{∗} | 29.18 | 19.33 |
| 47 | DRB1^{∗}03,DRB1^{∗}07;DRB3^{∗};DRB4^{∗} | 2.31 | 3.49 |
| 48 | DRB1^{∗}07,DRB1^{∗}15;DRB4^{∗};DRB5^{∗} | 2.20 | 29.21 |
| 49 | DRB1^{∗}03,DRB1^{∗}07;DRB3^{∗};DRB4^{∗} | 0.94 | 1.72 |
| 50 | DRB1^{∗}03,DRB1^{∗}15;DRB3^{∗};DRB5^{∗} | **0.73** | **3.27** |

In Table 1 above, the bolded section (donors 17, 18, 27, 30, and 50) shows cases in which the reactivity with KLH before and after thawing of the donor cells was significently different.

### Example 5: EpiScreen^{™}ex vivo T cell proliferation test

In order to identify the immunogenicity suppression mechanism according to the pegylated sites of insulinotropic peptides that are representative physiologically active protein or peptide, the T cell proliferative capacities of unbound native exendin-4 and unbound CA exendin-4, CA exendin-4 (CA Exendin-4-PEG(inter)) pegylated at the lysine residue, and the native exendin-4 (Exendin-4-PEG(N-term)) pegylated at the N-terminus were campared. At this time, since the CA exendin-4 does not have N-terminal residue that can be pegylated, the N-terminal pegylated CA-exendin-4 was not prepared for the CA exendin-4.

For T cell proliferation test, the peripheral blood mononuclear cells (PBMC) of the donor were thawed to measure the cell number and viability. Cells were diluted to 4-6×10⁶ cells/ml in AIM-V culture medium. After dispensing the cells of each donor in 24-well culture plates, the test samples were added to a final concentration of 50*µ*g/ml (n=3). The antigen peptide KLH treated group was placed to indentify the reproducility of each donor cell. All the test groups and the control groups were cultured at 37°C and 5% CO₂ incubator condition for 8 days. A part of the cells was taken on the 5th, 6th, 7th and 8th day and transferred to the 96-well culture plates to measure the cell proliferation rate. For the measurement of the cell proliferation rate, 0.75µi [³ H]-Thymidine (Perkin Elmer Buckinghamshire, UK) was added per well and cultured for 18 hours, and the cells were then collected with the 96-well filter plates using TomTec Mach III cell collecting device.

The radioactivity of each well (count per minute, cpm) was measured using 1450 Microbeta Wallac Trilux Liquid Scintillation Counter (Perkin Elmer Buckinghamshire, UK). The results have been determined based on the experimental thresholds of simulation index (SI) that two or more SI (SI≥2, p <0.05) was positive. In the case of including the boundary values corresponding to SI≥1.9, it was separately indicated as (P*). As a result, the CA exendin-4 and exendin-4 exhibited positive in 12% and 10% of donors, respectively. However, the CA exendin-4 pegylated to the internal residue of the peptide exhibited negative in all donors. On the other hand, the exendin-4 pegylated to the N-terminus exhibited positive in 6% of donors. Accordingly, if the pegylation was made to the internal lysine residue of the peptide rather than the N-terminus, the immunogenicity of the peptide was significantly inhibited (Table 2). Table 2 shows T-cell proliferation and interleukin-2 (IL-2) secretion.

**[Table 2]**

| | **CA Exendin-4** | **Exendin-**4 | **CA Exendin-4-PEG (inter)** | **Exendin-**4-PEG (N-term) | **Humanise d** A33 | **KLH** |
|---|---|---|---|---|---|---|
| **Donor 1** | PE | E | | | | PE |
| **Donor 2** | | | | | | E |
| **Donor 3** | | | | | E | PE |
| **Donor 4** | | | | P | E* | PE |
| **Donor 5** | | | | | | PE |
| **Donor 6** | | | | | | PE |
| **Donor 7** | | | | E* | P*E | PE |
| **Donor 8** | | | | | | P |
| **Donor 9** | PE* | PE | | | E* | PE |
| **Donor 10** | | | | | | P |
| **Donor 11** | | | | | | PE |
| **Donor 12** | | | | | | PE |
| **Donor 13** | | | | PE | | PE |
| **Donor 14** | | | | | | E |
| **Donor 15** | | | | | | P |
| **Donor 16** | | PE | | PE* | | PE |
| **Donor 17** | P | PE | | | PE | PE |
| **Donor 18** | | | | | PE* | PE |
| **Donor 19** | | | | | | PE |
| **Donor 20** | | | | | | PE |
| **Donor 21** | | E | E | | PE | PE |
| **Donor 22** | | | | | PE | PE |
| **Donor 23** | E | | | | | PE |
| **Donor 24** | | | | | | PE |
| **Donor 25** | | | | | | PE |
| **Donor 26** | | | | | | P*E |
| **Donor 27** | PE | P*E | | | | E |
| **Donor 28** | | | | | | PE |
| **Donor 29** | PE* | | | | | PE |
| **Donor 30** | | | | | | P |
| **Donor 31** | | | | | | PE |
| **Donor 32** | | | | | | PE* |
| **Donor 33** | | | | | P*E | P*E |
| **Donor 34** | | | | | | PE |
| **Donor 35** | | | | | | PE |
| **Donor 36** | | | | | PE | PE |
| **Donor 37** | | | | | | PE |
| **Donor 38** | | | | | | P |
| **Donor 39** | | | | | | PE |
| **Donor 40** | | | | | E* | PE |
| **Donor 41** | | | | | P*E | PE |
| **Donor 42** | | | | | | P |
| **Donor 43** | PE | | | | PE | PE |
| **Donor 44** | | | | | PE | PE |
| **Donor 45** | | | | | | PE |
| **Donor 46** | | | | | P*E | PE |
| **Donor 47** | | | | | | PE |
| **Donor 48** | | PE | | | | PE |
| **Donor 49** | | | | | | |
| **Donor 50** | | E | | | | PE |
| % Proliferation | 12 | 10 | 0 | 6 | 22 | 92 |
| **ELISpot** % | 12 | 16 | 2 | 6 | 30 | 86 |
| **Proliferation and** ELISpot % | 10 | 10 | 0 | 4 | 22 | 80 |
| **Correlation** % | 83 | 100 | N/A | 67 | 100 | 87 |

Table 3 shows the strength and frequency of T-cell proliferation resonse (including SI≥1.9 boundary value).

**[Table 3]**

| | **Mean SI** | **Standard Deviation** | **Frequency of Response** (%) |
|---|---|---|---|
| **CA** Exendin-4 | 2.09 | 0.2 | 12 |
| **Exendin**-4 | 2.68 | 1.46 | 10 |
| **CA** Exendin-4-PEG(inter) | N/A | N/A | 0 |
| **Exendin-4-PEG(N-term)** | 2.33 | 0.17 | 6 |
| **Humanised** A33 | 2.17 | 0.28 | 22 |
| **KLH** | 5.16 | 3.94 | 92 |

The above-described results suggest that the immunogenicity of the physiologically active protein or peptide bound to the non-peptidyl polymer, particularly PEG, through internal residue other than the terminal of the physiologically protein or peptide is inhibited.

### Example 6: EpiScreen^{™}ex vivo interleukin-2 (IL-2) secretion test

In order to identify the immunogenicity suppression mechanism according to the pegylated sites of insulinotropic peptides that are representative protein or peptide, the IL-2 secretory capacities of the unbound exendin-4 and the peglyated exendin-4 of Example 5 was compared and measured using donor cells and the samples, which are the same as in EpiScreenTM T cell proliferation assays. The anti-interleukin-2 antibody (R & D Systems, Abingdon, UK) was bound to ELISpot plates (Millipore, Herts, UK). The plate was washed three times with PBS (phosphate-buffered saline), and then PBS, supplemented with 1% bovine serum albumin, was added and reacted. After washing with AIM-V culture medium, the donor cells diluted with AIM-V medium (4-6×10⁶ cells/ml) were dispensed per 100 *µ*ℓ/well. The test sample was added each 50*µ*ℓ (n=6) so that the final concentration is 50 *µ*g/ml(n = 6). After culturing for 8 days, biotinylated IL-2 detection antibody and streptavidin-AP (R & D Systems, Abingdon, UK) were bound sequentially to ELISpot plates and then BCIP/NBT (R&D Systems, Abingdon, UK) was added to the plates to express a spot. The reaction was completed by washing with distilled water and then the plate was dried. Spots per well (spw) were scanned and analyzed using Immunoscan Analyser. The results of the activity measurement test of *ex vivo* T cells were determined based on the experimental threshold of stimulation index (SI) that two or more SI (SI≥2, p <0.05) was positive. In the case of including the boundary values corresponding to SI≥1.9, it was separately indicated as (P*). As a result, the CA exendin-4 and exendin-4 exhibited positive in 12% and 16% of donors, respectively. However, the CA exendin-4 pegylated to the internal residue of the peptide exhibited positive only in 2% of donors.. On the other hand, the exendin-4 pegylated to the N-terminus exhibited positive in 6% of donors. Accordingly, if the pegylation was made to the internal lysine of the peptide rather than the N-terminus, the immunogenicity of the peptide was significantly inhibited (Tables 2 to 4). Table 4 shows the strength and fequency of interleukin-2 (IL-2) secretion response of T cells (including SI≥1.9 boundary value).

**[Table 4]**

| | **Mean SI** | **Standard Deviation** | **Frequency of Response** (%) |
|---|---|---|---|
| **CA** Exendin-4 | 2.18 | 0.23 | 12 |
| **Exendin**-4 | 2.22 | 0.19 | 16 |
| **PEG-CA** Exendin-4 | 2.35 | N/A | 2 |
| **3.4K PEG(N-term) Exendin-4** | 2.09 | 0.17 | 6 |
| **Humanised** A33 | 2.24 | 0.43 | 30 |
| **KLH** | 3.79 | 1.84 | 86 |

The above-described results suggest that the immunogenicity of the physiologically active protein or peptide bound to the non-peptidyl polymer, particularly PEG, through internal residue other than the terminal of the physiologically protein or peptide is inhibited.

### Example 7: Production of antibodies against long-acting insulinotropic peptides in normal rats

The conjugates in which the CA exendin-4 was linked to immunoglobulin Fc fragment via PEG prepared in Example 3 were administrated subcutaneously to a normal Sprague Dawley rat once a week for 26 weeks (low, mild or high dosage), and then placed during the recovery period of 4 weeks (n = 40~60/group). The blood was collected before and during administration, at the 13rd, 19th and 26th week, and at the end of the recovery period, the serum was separated from this. It was determined on whether to produce the antibodies against the insulinotropic peptide.

As a result, among 160 subjects administered with a drug, the antibodies were detected in only two objects after the recovery period of 13 weeks. However, these antibodies were confirmed to be not neutralizing antibodies for the drug (Table 5). Table 5 shows the production of the antibody at 26-week administration in rats (SD Rat).

**[Table 5]**

| Group | Dose | n/group | Positive | Time point | % positive | Neutralizing Ab |
|---|---|---|---|---|---|---|
| 1 | vehicle | 60 | 0 | - | 0 | 0 |
| 2 | low dose | 40 | 1 | Week 13 | 2.5 | 0 |
| 3 | mid dose | 40 | 0 | - | 0 | 0 |
| 4 | high dose | 60 | 1 | recovery | 1.6 | 0 |
| Total | | 200 | 2 | | 1.0 | 0 |

### Example 8: Production test of antibodies against persistent insulin secretion peptide in Cynomolgus monkey

The conjugates in which the CA exendin-4 was bound to immunoglobulin Fc fragment via PEG prepared in Example 3 were subcutaneously administered to Cynomolgus monkey once a week for 26 weeks, and then placed during the recovery period of 4 weeks (n=8~12/group). The blood was taken before and during administration, at the 12th, 19th and 26th week, and at the end of the recovery period, the serum was separated from this. It was determined whether to produce the antibodies against the insulinotropic peptide.

As a result, no production of the antibodies in all subjects was detected (Table 6). Table 6 shows production of antibodies at the 26-week administration in Cynomolgus monkey.

**[Table 6]**

| Group | Dose | n/group | Positive/ total | % positive |
|---|---|---|---|---|
| 1 | vehicle | 12 | 0 | 0 |
| 2 | low dose | 8 | 0 | 0 |
| 3 | mid dose | 8 | 0 | 0 |
| 4 | high dose | 12 | 0 | 0 |
| Total | | 40 | 0 | 0 |

### Example 9: Detection of the anti-drug antibody in the blood and evaluation of the neutralizing capacity

In order to detect whether the conjugate of Example 3 has produced an anti-drug antibody (ADA) in the body of rat or Cynomolgus monkey, the conjugate was examined by the bridging ELISA method. The biotinylated conjugate of Example 3 was bound to the 96-well microplate in which streptavidin was coupled to the bottom thereof, and washed with water. Digoxigenin(DIG)-labeled conjugate of Example 3 (hereinafter, HM11260C) was added along with the serum samples of rat or monkey to react and then washed with water. Then, the horseradish peroxidase-coupled anti-DIG antibody (anti-DIG-POD antibody) was added and developed by TMB substrate (3,3',5,5'-tetramethylbenzidine substrate).

Measurement sensitivity in the rat serum was 3.1 ng/ml, and the measurement sensitivity in monkey serum was 12.5 ng/ml. To evaluate the neutralizing capacity against HM11260C of detected anti HM11260C antibody, serum samples and HM11260C were added to the human GLP-1 overexpressed cell line (GLP-1R/CHO) and then the inhibiton rate of cAMP-induction was measured. The antibodies produced by only two of the 160 animals were confirmed to have no neutralizing ability.

The above-described results suggest that the immunogenicity of the physiologically active protein or peptide was decreased by binding a non-peptide linker and Fc fragment to the internal residue other than the terminal of the physiologically active protein or peptide, thus inhibiting the mechanism in which the desired peptide acts as an antigen. The results also support that, in the case of using the producing method as described above, the activation of T cells and the antibody production reaction in animals were significantly inhibited.

From the foregoing description, it will be understood by those skilled in the art that the present invention may be embodied in other specific forms without changing the technical spirit or essential characteristics of the invention. In this regard, the above-described embodiments are for illustrative purposes and should be understood to be not limited thereto. It should be interpreted as encompassing all changes or modified forms derived from the meaning and range and equivalents thereof of the appended claims rather than the foregoing detailed description.

<110>    HANMI PHARM. CO., LTD.

<120>    Method for decreasing immunogenicity of protein and peptide

<130>    OPA15095-PCT

<150>    KR 10-2014-0068660
<151>    2014-06-05

<160>    5

<170>    KopatentIn 2.0

<210>    1
<211>    21
<212>    PRT
<213>    Artificial Sequence

<220>
<223>    Alpha chain of insulin


<400>    1
Gly Ile Val Glu Gln Cys Cys Thr Ser Ile Cys Ser Leu Tyr Gln Leu
  1               5                  10                  15

Glu Asn Tyr Cys Asn
             20


<210>    2
<211>    30
<212>    PRT
<213>    Artificial Sequence

<220>
<223>    Beta chain of insulin


<400>    2
Phe Val Asn Gln His Leu Cys Gly Ser His Leu Val Glu Ala Leu Tyr
  1               5                  10                  15

Leu Val Cys Gly Glu Arg Gly Phe Phe Tyr Thr Pro Lys Thr
             20                  25                  30


<210>    3
<211>    39
<212>    PRT
<213>    Artificial Sequence

<220>
<223>    Exendin-4


<400>    3
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Pro Ser
         35


<210>    4

35
<211>    39
<212>    PRT
<213>    Artificial Sequence

<220>
<223>    Exendin-3


<400>    4
His Ser Asp Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35


<210>    5
<211>    37
<212>    PRT
<213>    Artificial Sequence

<220>
<223>    oxyntomodulin


<400>    5
His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr Lys Arg Asn
            20                  25                  30

Arg Asn Asn Ile Ala
        35

The following clauses set out further aspects of the present invention.

### [Clause 1]

A method for decreasing immunogenicity of a physiologically active protein or peptide as compared to that of a physiologically active protein or peptide to which a carrier is not bound, which comprises binding a carrier to the non-terminal, internal residue of the physiologically active protein or peptide.

### [Clause 2]

The method according to clause 1, wherein the carrier is selected from the group consisting of a polyethylene glycol, a fatty acid, a cholesterol, an albumin or a fragment thereof, an albumin-binding substance, a polymer having repeating units of a particular amino acid sequence, an antibody, an antibody fragment, a FcRn binding substance, an *in-vivo* connective tissue or a derivative thereof, a nucleotide, a fibronectin, a transferrin, an elastin-like polypeptide(ELP), a XTEN polypeptide, a carboxy-terminal peptide (CTP), a structure inducing probe (SIP), a saccharide and a high molecular weight polymer.

### [Clause 3]

The method according to clause 2, wherein the FcRn binding substance includes an immunoglobulin Fc region.

### [Clause 4]

The method according to clause 1, wherein the physiologically active protein or peptide and the carrier are bound via a linker interposed therebetween.

### [Clause 5]

The method according to clause 3, wherein the linker is a non-peptidyl linker.

### [Clause 6]

The method according to clause 4, wherein the non-peptidyl linker is selected from the group consisting of a polyethylene glycol, a polypropylene glycol, an ethylene glycol-propylene glycol copolymer, a polyoxyethylated polyol, a polyvinyl alcohol, a polysaccharide, a dextran, a polyvinyl ethyl ether, a biodegradable polymer, a lipid polymer, a chitin, a hyaluronic acid and a combination thereof.

### [Clause 7]

The method according to clause 4, wherein the physiologically active protein or peptide is bound to an immunoglobulin Fc region via a non-peptidyl polymer which is selected from the group consisting of a polyethylene glycol, a polypropylene glycol, an ethylene glycol-propylene glycol copolymer, a polyoxyethylated polyol, a polyvinyl alcohol, a polysaccharide, a dextran, a polyvinyl ethyl ether, a biodegradable polymer, a lipid polymer, a chitin, a hyaluronic acid and a combination thereof.

### [Clause 8]

The method according to clause 1, wherein the physiologically active protein or peptide is selected from the group consisting of an anti-obesity peptide, an insulinotropic peptide or an analog thereof, a leptin, an insulin, an insulin analog, a glucagon, a human growth hormone, a growth hormone releasing hormone, a growth hormone releasing peptide, an interferon, an interferon receptor, a colony stimulating factor, a glucagon-like peptide such as GLP-1, a GLP-1/glucagon dual agonist, a gastric inhibitory polypeptide (GIP), a G-protein-coupled receptor, an interleukin, an interleukin receptor, an enzyme, an interleukin binding protein, a cytokine binding protein, a macrophage activating factor, a macrophage peptide, a B cell factor, a T cell factor, a protein A, an allergy inhibitory factor, a cell necrosis glycoprotein, an immunotoxin, a lymphotoxin, a tumor necrosis factor, a tumor inhibitory factor, a metastasis growth factor, an alpha-1 antitrypsin, an albumin, an α-lactalbumin, a apolipoprotein-E, an erythropoiesis factor, a highly glycosylated erythropoiesis factor, an angiopoietin, a hemoglobin, a thrombin, a thrombin receptor activating peptide, a thrombomodulin, blood factors VII, VIIa, VIII, IX and XIII, a plasminogen activating factor, a fibrin-binding peptide, an urokinase, a streptokinase, a hirudine, a protein C, C-reactive protein, a renin inhibitor, a collagenase inhibitor, a superoxide dismutase, a platelet-derived growth factor, an epithelial cell growth factor, an epidermal growth factor, an angiostatin, an angiotensin, a bone growth factor, a bone stimulating protein, a calcitonin, an atriopeptin, a cartilage inducing factor, an elcatonin, a connective tissue activating factor, a tissue factor pathway inhibitor, a follicle stimulating hormone, a luteinizing hormone, a luteinizing hormone releasing hormone, a nerve growth factor, a parathyroid hormone, a relaxin, a secretin, a somatomedin, an insulin-like growth factor, an adrenocortical hormone, a glucagon, a cholecystokinin, a pancreatic polypeptide, a gastrin-releasing peptide, a cortincotropin releasing factor, a thyroid stimulating hormone, an autotaxin, a lactoferrin, a myostatin, a receptor, a receptor antagonist, a cell surface antigen, a virus-derived vaccine antigen, a monoclonal antibody, a polyclonal antibody, and an antibody fragment.

### [Clause 9]

The method according to clause 8, wherein the physiologically active protein or peptide is selected from the group consisting of an exendin-4, an exendin-4 derivative, a GLP-1 agonist, an insulin and a GLP-1/glucagon dual agonist.

### [Clause 10]

The method according to clause 9, wherein the exendin-4 derivative is an exendin-4 derivative in which the charge on the N-terminal of exendin-4 is modified, which is selected from the group consisting of an exendin-4 derivative in which N-terminal amine group of exendin-4 is deleted; an exendin-4 derivative in which N-terminal amine group of exendin-4 is substituted with hydroxl group; an exendin-4 derivative in which N-terminal amine group of exendin-4 is substituted with carboxyl group; an exendin-4 derivative in which N-terminal amine group of exendin-4 is modified with dimethyl group; and an exendin-4 derivative in which alpha carbon of N-terminal histidine residue of exendin-4 is deleted.

### [Clause 11]

The method according to clause 9 or 10, wherein the internal residue is a lysine residue at position 12 or 27 of the exendin-4 derivative in which N-terminal charge of exendin-4 is modified.

### [Clause 12]

The method according to clause 11, wherein the internal residue is a lysine residue at position 27 of the exendin-4 derivative in which N-terminal charge of exendin-4 is modified.

### [Clause 13]

The method according to clause 11, wherein the exendin-4 derivative in which the charge on the N-terminal of the exendin-4 is modified is an exendin-4 derivative in which alpha carbon of N-terminal histidine residue of exendin-4 is deleted.

### [Clause 14]

A composition, comprising a conjugate of a physiologically active protein or peptide in which a carrier is bound to the non-terminal, internal residue of a physiologically active protein or peptide, via a non-peptidyl linker, wherein the conjugate exhibits decreased immunogenicity as compared to that of the physiologically active protein or peptide to which the carrier is not bound.

### [Clause 15]

The composition according to clause 14, wherein the conjugate has decreased immunogenicity, which is a side effect of a long-acting preparation.

### [Clause 16]

The composition according to clause 14, wherein the non-peptidyl linker is a polyethylene glycol.

## Claims

1. A method for decreasing immunogenicity of a physiologically active protein or peptide as compared to that of a physiologically active protein or peptide to which an immunoglobulin Fc region is not bound, which comprises;
binding an immunoglobulin Fc region to the non-terminal, internal residue of the physiologically active protein or peptide,
wherein the physiologically active protein or peptide is an exendin-4, or an exendin-4 derivative in which the alpha carbon of the N-terminal histidine residue of exendin-4 is deleted.

2. The method according to claim 1, wherein the physiologically active protein or peptide and the immunoglobulin Fc region are bound via a linker interposed therebetween.

3. The method according to claim 2, wherein the linker is a non-peptidyl linker.

4. The method according to claim 3, wherein the non-peptidyl linker is selected from the group consisting of a polyethylene glycol, a polypropylene glycol, an ethylene glycol-propylene glycol copolymer, a polyoxyethylated polyol, a polyvinyl alcohol, a polysaccharide, a dextran, a polyvinyl ethyl ether, a biodegradable polymer, a lipid polymer, a chitin, a hyaluronic acid and a combination thereof.

5. The method according to claim 4, wherein the molecular weight of the polyethylene glycol ranges from 1 to 20 kD.

6. The method according to claim 1, wherein the internal residue is a lysine residue at position 12 or 27 of the exendin-4 or the exendin-4 derivative.

7. The method according to claim 6, wherein the internal residue is a lysine residue at position 27 of the exendin-4 or the exendin-4 derivative.

8. Use of a pegylated immunoglobulin Fc region as a carrier in a method decreasing immunogenicity of a physiologically active protein or peptide as compared to that of a physiologically active protein or peptide to which a carrier is not bound, wherein the use comprises binding the carrier to the non-terminal, internal residue of the physiologically active protein or peptide,
wherein the physiologically active protein or peptide is an exendin-4, or an exendin-4 derivative in which the alpha carbon of the N-terminal histidine residue of exendin-4 is deleted;
wherein the carrier is bound to the non-terminal, internal residue of the physiologically active protein or peptide via a non-peptidyl linker, and the non-peptidyl linker is a polyethylene glycol.
